# EUROPEAN PATENT APPLICATION

(11) **EP 0 705 841 A1**
(43) Date of publication of application: **10.04.1996**
(21) Application number: 94830474.6
(22) Date of filing: 05.10.1994
(51) Int. Cl.: C07K 7/06, C07K 7/08, C07K 14/00, A61K 38/08, A61K 38/10, A61K 38/16

(54) **Peptides havng antigenic activity against hepatitis A virus (HAV)**

(71) Applicant: SORIN BIOMEDICA S.p.A., I-13040 Saluggia (Vercelli) (IT); CONBIOTEC - CONSORZIO PER LE BIOTECNOLOGIE, I-25123 Brescia (IT)
(72) Inventor: Bonelli, Fabrizio, I-15033 Casale Monferrato, Alessandria (IT); Primi, Daniele, I-25100 Brescia (IT); Albertini, Alberto, I-25122 Brescia (IT); Mattioli, Sonia, I-31010 Moriago della Battaglia, Treviso (IT)
(74) Representative: de Simone, Domenico

(57) **Abstract**

A peptide with antigenic activity against HAV, having an amino acid sequence, that cannot be deducted from the amino acid sequence of HAV codified proteins, at least comprising the sequence, -D-V-M-Y-L-K (Seq.ID:35).

## Description

This invention relates to peptides with amino acid sequences not correlated with the sequence of Hepatitis A Virus (HAV) codified proteins, having an HAV antigenic activity, to be used for hepatitis A diagnosis and immunotherapy purposes.

It is very difficult to isolate and obtain in vitro such a purified HAV amount that could be used for diagnosis and immunotherapy purposes, as well as for starting material for the cloning of the viral genome.

Peptides according to the invention overcome the problems as above, by identifying peptide sequences, that are able to mimic an HAV viral epitope. Moreover they may be easily chemically synthesized in an adequate amount to be used on a large scale in diagnostic assays and immunogenic compositions.

Peptides according to the invention were selected from chemical hexapeptide libraries and then synthesized through chemical processes. However, the peptides can be obtained using any process known by the skilled in the art.

The hexapeptide library was screened with a monoclonal antibody obtained from mice immunized with HAV isolated human faeces and in vitro cultivated. Surprisingly, the authors identified a linear peptide having an HAV not related amino acid sequence, that was able to mimic a viral conformational epitope, i.e. a not linear, not consecutive amino acid sequence.

The peptide was synthesized on a solid phase both in monomer (PM) and tetramer (P4) form. The P4 form showed to react to a larger extent with the monoclonal antibody than the PM form. Further studies led to optimize the amino acid sequence, that is able to mimic the HAV antigenic activity.

The peptides according to the invention are recognized by anti-HAV human polyclonal antibodies and therefore they are suitable to be used in diagnosis and irnunotherapy kits. The use in an immunotherapy kit was also confirmed through mice immunization experiments, that showed a specific immunogenic ability of said peptides. Accordingly, an HAV specific immunity response in vivo could be obtained by immunizing animals using the peptides according to the invention.

Therefore, it is an object of the invention a peptide having an HAV antigenic activity and an amino acid sequence that cannot be deducted from the amino acid sequence of HAV codified proteins, at least comprising the sequence identified as SEQ ID No. 35.

Preferably, the peptide according to the invention comprises the sequence identified as SEQ ID No. 37.

Alternatively, the peptide according to the invention comprises the SEQ ID No. 46.

According to a preferred embodiment of the invention, the peptide comprises a spacer arm, more preferably the spacer arm comprises at least three glycine residues linked to the peptide either C-terminal or N-terminal by means of peptide bonds.

Further according to the invention, the peptide is as a monomeric linear form; preferably the sequence is repeated more than once, preferably twice, spaced at least by one glycine residue, more preferably three or five glycine residues.

Alternatively the peptide is as a polymeric form, preferably as a tetrameric form, according to the formula as follows: 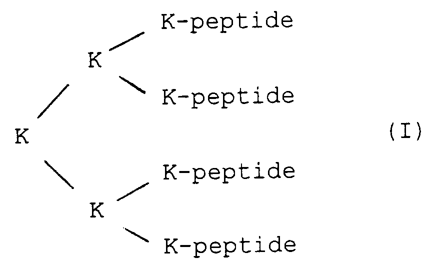
wherein K is a lysine residue.

It is also an object of the invention a composition comprising the peptides of the invention, to be used as a specific reactant for diagnostic kits to detect antibodies against HAV in a sample.

It is a further object of the invention an HAV immunogenic composition, comprising the peptides of the invention, to be used as a vaccine or as a starting material for anti-HAV immune sera.

This invention is described in the following with reference to some specific, but not limiting embodiments and to some tests showing the peptide activity. In the following, reference is made to the annexed figures, wherein:
Fig. 1 shows the 8F4H9B-HRP monoclonal antibody reactivity against the PM peptide in solid phase at various concentrations;
Fig. 2 shows the 8F4H9B-HRP monoclonal antibody reactivity against the PM peptide (Table 1, no. 35) as well as four mutant peptides in solid phase (Table 1, peptides no. 36, 1, 4 and 17);
Fig. 3 shows the 8F4H9B-HRP monoclonal antibody reactivity against the P4 peptide in solid phase at various concentrations;
Fig. 4 shows the reactivity of antibodies that are not related to the P4 peptide, in solid phase;
Fig. 5 shows the binding inhibition of 8F4H9B-HRP monoclonal antibody to HAV with the P4 peptide, in a solution assay;
Fig. 6 shows the binding inhibition of the P4 peptide to HAV with the 8F4H9B-HRP monoclonal antibody or anti-HAV human antibodies, in a solid phase assay;
Fig. 7 shows the specific binding inhibition of the 8F4H9B-HRP monoclonal antibody to the P4 peptide with HAV, in a solid phase assay; the control was carried out by inhibiting the binding of a not related anti-HCV monoclonal antibody to antibodies anti-murine Igs with HAV, in a solid phase assay;
Fig. 8 shows the reactivity of the ascitic fluid, obtained through immunizing Balb/C mice with both the P4 peptide and a not related peptide, against the P4 peptide adhering to a solid phase;
Fig. 9 shows the reactivity of the fraction, obtained through precipitating the anti-P4 peptide ascitic fluid with 18% w/v of Na₂SO₄, against the same P4 peptide, as well as HAV adhering to a solid phase;
Fig. 10 shows the reactivity of the fraction, obtained through precipitating the anti-P4 peptide ascitic fluid with 18% w/v of Na₂SO₄, against HAV adhering to a solid phase, using as tracers anti-mouse Igs or an anti-mouse K chain monoclonal antibody;
Fig. 11 shows the reactivity of the fractions, obtained through precipitating the anti-P4 peptide ascitic fluid, as well as an anti-not related peptide ascitic fluid with 18% w/v of Na₂SO₄ to HAV adhering to a solid phase, using as tracers a monoclonal antibody anti-murine K chain at two different concentrations (panels A and B);
Fig. 12 shows the reactivity of peptides identified through screening the second sublibrary (Table 2), in tetramer form and adhering to the solid phase at 10 µg/ml, with the 8F4H9B-HRP monoclonal antibody;
Fig. 13 shows the reactivity of the peptide 1 (Table 2) in tetramer form adhering to the solid phase, with the 8F4H9B monoclonal antibody;

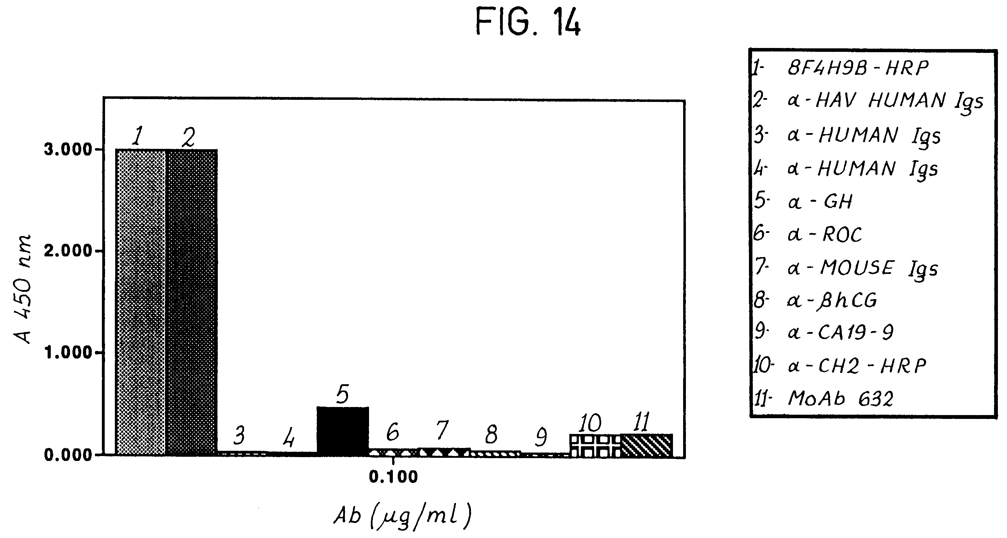

Fig. 14 shows the reactivity of the peptide 1 (Table 2) in tetramer form adhering to the solid phase at 10 µg/ml, against various not related antibodies;
Fig. 15 shows the reactivity of the peptide 1 (Table 2) in monomer form adhering to the solid phase after having been diluted in 50 mM pH 9.6 carbonate buffer, and incubated for 2 hours at 37°C, with the 8F4H9B monoclonal antibody;
Fig. 16 shows the reactivity of the peptide 1 (Table 2) in monomer form adhering to the solid phase after having been diluted in 50 mM pH 9.6 carbonate buffer, and incubated overnight at 4°C, with the 8F4H9B monoclonal antibody;

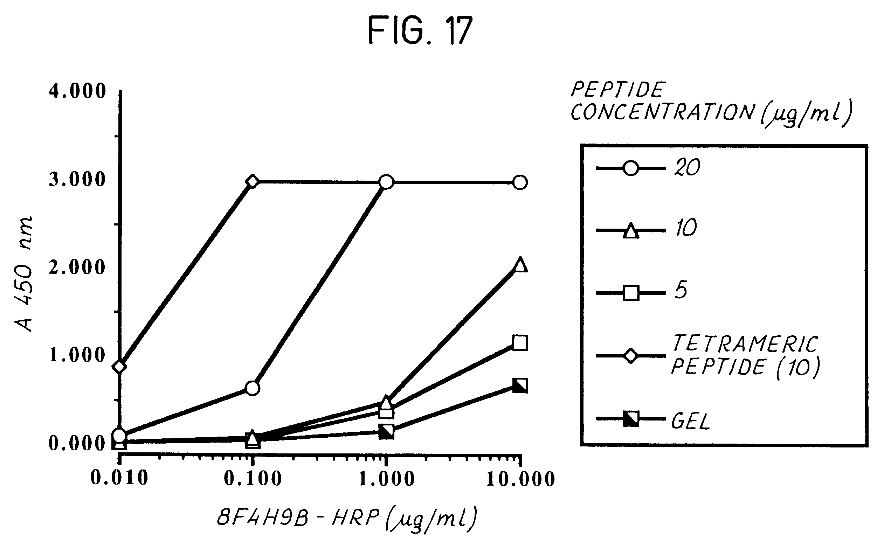

Fig. 17 shows the reactivity of the peptide 1 (Table 2) in monomer form adhering to the solid phase after having been diluted in a 50/50 trifluoroethanol/H₂O mixture and incubated overnight at room temperature, with the 8F4H9B monoclonal antibody;
Fig. 18 shows the reactivity of 1G, 3G and 5G peptides adhering to the solid phase at 10 µg/ml after having been diluted in a 50/50 trifluoroethanol/H₂O mixture and incubated overnight at room temperature, with the 8F4H9B monoclonal antibody;
Fig. 19 shows the reactivity of the listed peptides adhering to the solid phase at 0.1 µg/ml concentration, after having been diluted in a 50/50 thrifluoroethanol/H₂O mixture, with a not related antibodies at 0.1 µg/ml;
Fig. 20 shows the reactivity of the listed antibodies adhering to the solid phase, after having been diluted in a 50/50 trifluoroethanol/H₂O mixture, with the 8F4H9B monoclonal antibody; and
Fig. 21 shows the reactivity of 1D in tetramer form and P4 peptides adhering to the solid phase after having been diluted in 50 mM pH 9.6 carbonate buffer, with the 8F4H9B monoclonal antibody.

### PREPARATION OF THE HEXAPEPTIDE LIBRARY

The "Selectide" peptide library preparation method allows to obtain millions of resin particles, each one containing a single peptide. Accordingly, the concerned full collection shall reflect quite the whole number of possible sequences in approximatively equimolar amounts.

The synthesis procedure is based on coupling a single amino acid residue with a resin, which is obtained through mixing resins with 19 different amino acids in equal amounts. The twentieth amino acid, i.e. the cysteine, is normally excluded from the synthesis, in order to prevent any reactivity problems of its sulphidryl group. After having been coupled with a single amino acid, the resins are mixed, subdivided again and further coupled to other amino acids. The process is repeated many times until a peptide with the desired lenght is obtained. At the end of the synthesis process, each particle consists of only one peptide sequence, as each resin particle was coupled to single amino acids.

A suitable solid carrier when using the Selectide technique is the "PAM" resin, a polystirene based resin (1), wherein the binding with the peptide may be broken only when very strong acids, such as hydrofluoric acid, or mixtures of hydrobromic acid and trifluoroacetic acid, are used. Accordingly, a selective splitting of the protection groups of the amino acid side chains could be carried out, without interfering on the peptide/resin complex. The synthesized peptides could be spaced from the resin by a spacer arm, e.g. comprising three glycines, in order to minimize any interaction with the solid carrier, if any, and to allow a better sequence exposure.

Only one amino acid sequence shall correspond to each resin particle; therefore, the number of resin particles that were used in the synthesis process, must necessarily correspond to the number of different peptides, of which the library potentially consists. In the case of an hexapeptide library, 19⁶ = 47.045.881 different peptides could be foreseen; by assuming that at least 10 resin particles are necessary for each peptide, at least 17.2 grams of PAM resin are requested. The calculation is based on the max. diameter of the particles, according to the Gauss diameter distribution, indicated by the Supplier, and, accordingly, the number of the actually present particles is higher than the theoretical amount as above.

In order to carry out the synthesis process, the chemistry of the amino acids, protected at the alpha position of amino groups by a Fmoc (9-fluorenylmethoxycarbonyl) group, that could be removed by means of a solution of 20% piperidine in N,N,dimethylformamide (DMF), could be applied. The side chains are protected by groups, that could be removed by TFA (trifluoroacetic acid), and the carboxyl group could be activated previously as an active ester. The synthesis reactions could be monitored by means of the Kaiser's Test (2); any not completed reaction must be repeated. After each coupling, resin aliquots are collected, deprotected at the alpha positions of amino groups, mixed and divided again in 19 aliquots.

At the last coupling, resins are kept separately, and the amino acid side chain protecting groups are deprotected, maintaining as intact the resin-peptide complex. Nineteen sublibraries are obtained through this procedure, the only known sequence of which is the N-terminal amino acid sequence. The sublibraries are characterized through an amino acid analysis and tested with the monoclonal antibody, which must have a label, allowing to detect the binding to the resin particles. A detecting system comprises to conjugate the antibody to enzymes, such as peroxydase or alkaline phosphatase. The addition of the corresponding substrate allows to detect the reaction. Another detecting system comprises a conjugation to fluorescent derivatives, e.g., fluorescein. The interesting bound particles are separated through a cytofluorometry procedure. Also not conjugated molecules could be used; however, in this case, the reaction detection is to be carried out by means of a suitably specific molecule reactant; e.g., a murine antibody could be detected with an antibody against murine Igs. Also the molecules could be anchored to magnetic balls and the complex detaching carried out using a magnet.

### MATERIALS

All Fmoc protected, previously activated as esters, amino acids, the Boc-GLY-PAM (terbuthyloxycarbonyl-glycine-PAM) at an effective function concentration of 0.3 mmol/g, HOBT (1-hydroxybenzotriazole) were supplied by NovaBiochem (Cambridge, U.K.). DMF, piperidine, TFA, organic solvents and salts were supplied by Carlo Erba (Milan, Italy). Hydrocloric acid and phenyl-isothiocyanate are available Pierce products on the market. BSA (bovine serum albumin) was supplied by Sigma (St.Louis, Mo, US).

### METHODS

The library was defined using the RaMPS half-manual synthesizer (DuPont, Germany), that allows to synthesize 25 peptides at the same time. The unit consists of an oscillating plate equipped with 25 cartridges, to which a suction system is connected.

The synthesized library shows the formula as follows: X1-X2-X3-X4-X5-X6-G-G-G-PAM, wherein X(1-6) are amino acid residues; G-G-G is a three glycine residue spacer arm; and PAM is the resin. More glycine residues or different amino acid residues could be used.
5 g of Boc-Gly-PAM with 0.3 mmol/g as function level were suspended in methylene chloride (DCM), filtered and treated with a mixture 1:1 TFA/DCM under slow stirring for 30 minutes at room temperature in order to split the Boc group. The resin was washed five times with DCM and treated with a mixture 1:10 TFA/DCM for 10 minutes at room temperature under slow stirring. After having been washed three times with DCM and once with DMF, the resin was reacted with a 5 X excess of Fmoc-Gly-OPfp (OPfp=pentafluorophenyl) in HOBT, 0.33 M/ DMF, at room temperature under stirring, until the reaction was completed. The reaction was monitored trough the Kaiser's test. When the coupling was obtained, the resin was washed three times with DMF, twice with methanol, three times with DMF and subjected to a further Fmoc-Gly-PAM coupling under the same conditions as above. After having the Fmoc group splitted, the resin was suspended in a 60:40 DCM/DMF mixture and, under stirring at room temperature, was subdivided in 19 portions, that were placed in the 19 cartridges of the RaMPS synthesizer. Each resin portion was added with a 5 X excess of a different amino acid in 0.33 M HOBT/DMF. All the amino acids were protected with Fmoc group at the alpha positions of the amino group, previously activated at the carboxyl group as pentafluorophenyl esters, and as hydroxydihydrobenzotriazine esters, when serine and threonine were involved. The side chains protecting groups were as follows: Mtr(4-methoxy-2,3,6-trimethylbenzene-sulfonyl), for arginine; Trt, for asparagine, glutamine and histidine; tBu (tert-butyl), for tyrosine, serine and threonine; and OtBu, for the aspartic and glutamic chains.

The reactions were carried out until completed at room temperature under stirring. At this point, the portions were washed with DMF, collected and treated with a mixture of piperidine, 20%, in DMF at room temperature, under rotation. Then, the mixtures were washed three times with DMF, twice with CH₃OH and three further times with DMF. The resin was then suspended in the 60:40 DCM/DMF mixture, stirred for 20 minutes at room temperature and subdivided in 19 portions. The same procedure as above was repeated up to the last amino acid coupling, when the last 19 portions were kept separated. The amino group at alpha position was detached with piperidine 20%, in DMF for 20 min. at room temperature and then washed with DMF, glacial acetic acid, t-amyl alcohol and ethyl ester were carried out. Then the fractions were vacuum dried for 2 hours. Finally the sublibraries were subjected to cut in order to remove the side chain protecting groups, using a mixture, comprising TFA 85%, EDT (1,2-ethanedithiol) 2.5%, phenol 5%, thioanizole 5% and H₂O 2.5%, overnight at room temperature.

The subsequent sublibraries with N-terminal amino acids, identified in the following, were synthesized trough the same procedure.

### KAISER'S TEST

This test detects free amino groups on the resin and is to be carried out using three solutions as follows:
- solution "A": 5% ninhydrin in ethanol;
- solution "B": 400% phenol in ethanol (w/v);
- solution "C": 20 µl KCN in piridine.

The test, involving a reference white color to be obtained, was carried out by adding 40 µl of the A solution, 50 µl of the B solution and 100 µl of the solution to a small resin amount, followed by heating the mixtures to 100°C for 5 min. After cooling, the reference white color was compared to the resin colour; should both reference and sample colors were the same, no free amino groups on the resin were present; on the contrary, a different color detection meant that free amino groups were present.

The peptides were assayed in its monomer or polymer (tetra- or octamer) form (3), in order to increase its activity. Actually, monomer peptides could show some adhesion problems to the solid phases.

### PREPARATION OF ANTI-HAV MONOCLONAL ANTIBODIES

The hexapeptide library was assayed with the HAV specific monoclonal antibody 8F4H9B, that was obtained after mice immunization with virus which was isolated from human faeces and in vitro grown on Vero cells, as described herebelow.

Vero cells were grown in a culture medium, containing 5% FCS, up to confluence and then infected with a virus stock maintained at -80°C. The virus containing supernatant was collected each week for appr. three months and the virus was concentrated through molecular filtration. The concentrated product was precipitated with 30% saturated ammonium sulphate, resuspended in PBS and then dialyzed against PBS. The virus was inactivated by treatment with formalin. After reactivation, the virus was further purified by ultracentrifugation on a saccarose gradient. The fraction containing the highest amount of virus was dialyzed, in order to remove the saccarose excess and the protein contents defined, using the BCA technique.

Three female Balb/C mice were immunized by three subcutaneous injections of 50 µg of virus preparation in Freund's adjuvant at 15 day intervals. The antibody response was monitored, using blood samples from the retro-orbital plexus. Serum samples were assayed for the antibody contents using a competitive RIA dosing system (AB-HAVK).

Then spleen cells of the mouse, showing the highest contents of specific antibodies, were fused with SP2/0 myeloma cells. Supernatants of resulting hybridomas were assayed for the presence of antibodies against HAV through an indirect ELISA assay, wherein a solid phase with adsorbed HAV and HRP comjugated murine IgG-anti as labels were used.

The ELISA assay positive hybridoma reactivity was confirmed through a competitive RIA (AB-HAVK) assay. Positive hybridoma after both assays, showing the highest ability to inhibit the human radiolabelled polyclonal antibody, was cloned on 96 well trays. Clones were analyzed as previously described and the 8F4H9B clone was selected on the basis of both the reactivity and the better cell growth.

The selected clone was assayed for isotype of produced atibodies using a Sigma kit. The obtained subtype was IgG1K.

3x10⁶ cells of the 8F4H9B clone were intraperitoneally injected to ten BALB/C mice. The obtained ascites were collected in a single pool and then the IgG purified with protein A.

### LIBRARY SCREENING

The resulting 19 sublibraries were washed twice with a solution of PBS/BSA 0.1%, and the supernatant pH was checked to be approx. 7. Test tubes, containing the diluted sublibraries in 1 ml of PBS/BSA 0.1% were stirred for one hour at room temperature. The resin was separated from the supernatant through centrifuging and incubated with 1 ml of a 10 ng/ml solution of the HRP conjugated 8F4H9B antibody, diluted in PBS/BSA 0.1%, under rotating stirring, for 1 hour at room temperature. After one hour, resins were separated from the supernatant through centrifuging and washed five times with PBS/Tween 0.1%. Each portion was added with 500 µl of a solution, comprising 45 mg of OPD (O-phenylendiamine) in 15 ml of phosphocytrate buffer 25mM and 10 µl of H₂O₂. After having blocked the reaction with a twofold volume of H₂SO₄ 1N, the absorbance was readed and the sublibrary showing the highest absorption at 492 nm was defined as positive.

### PEPTIDE SYNTHESIS

The peptides were synthesized in the monomer and tetramer form using a continuous peptide flow, automatic MilliGen 9050 synthesizer (Millipore, Milford, U.S.). All amino acids were protected with Fmoc at the alpha position of the amino group, previously activated at the carboxylic residue as esters and protected at reactive side chains. All coupling reactions were carried out in HOBT 0.33M/DMF; washings were performed with DMF and the amino group at alpha position was released using piperidine 20%, in DMF. When the synthesis was completed, peptides were splitted from the resin using the conventional reactants as described in the literature. Peptides were precipited, using glacial diethyl ether and freeze-dryed, after having been dialyzed with a solution of glacial acetic acid 5% in water.

Raw peptides showed a purity level of 95% when analyzed with RP-HPCL. Peptides were characterized and identified as the specifically concerned peptides through amino acid analysis.

### AMINO ACID ANALYSIS

Peptide library resins were hydrolyzed in liquid phase with HCl 6N, containing phenol 0.1%, for 24 hours at 110°C. The supernatant was dried and the amino acids derived using PITC (phenylisothiocyanate)(4), analysed and quantified using RP-HPLC.

The peptide amino acid analysis was carried out through hydrolyzing the peptides with gaseous HCl, containing phenol 0.1%, for 2 hours at 157°C. The obtained amino acids were derived, using PITC, analyzed and quantified using RP-HPLC.

### ELISA ASSAY

The solid phase was obtained on PVC trays (Nunc), that were added with 200 µl of a peptide solution at various concentrations in a carbonate buffer 50 mM, pH 9.6. Trays were incubated overnight at 4°C when added with the monomer peptide solution, and for 2 hours at 37°C when added with the tetramer peptide solution.

Trays were saturated with 300 µl of a solution of gelatin 2%, in Tris-HCl, 0.1M, pH 7.5, for 1 hour at room temperature. Antibodies were diluted in a solution, comprising PBS, EDTA 0.03%, Triton-X100 0.2%, Tween 20 0.2% and BSA fraction V 3.2%; and incubated for 3 hours at 37°C. Trays were washed four times with an ET-System Sorin automatic washer, using PBS/Tween 20 0.1%, and the reaction detected by adding 100 µl of a chromogen and a substrate (Sorin DEIA KIT). The reaction was blocked after 30 min. by adding 200 µl of H₂SO₄ 1N, and the absorbance read at 450 nm.

### INHIBITION TEST

Trays to which HAV was adhered (ETI-AB-HAVK-2, Sorin) were used for the inhibition test. The peptide was serial diluted, starting from 5 mg/ml, in a solution of 0.05 µg/ml of the 8F4H9B-HRP antibody in the buffer that was used for the ELISA assay. 200 µl of each sample were centrifuged and incubated for 3 hours at 37°C.

Inhibitions tests against the virus were carried out by incubating 200 µl of a culture medium, that was diluted in series, using a solution of 0.05 µg/ml of 8F4H9B-HRP antibody and 0.05 µg/ml of human polyclonal antibodies against HAV into the solution of each sample, as above, for 3 hours at 37°C. Washings and reaction detecting procedures were the same as described for the ELISA assay.

### BALB/C MICE IMMUNIZATION WITH THE P4 PEPTIDE

Mouse immunizations with tetramer P4 peptide both in free form and conjugated with hemocyanine were carried out as follows:
100 µg of each preparation were emulsified with Freund's complete adjuvant for the first immunization and subcutaneously injected into the leg plantar. The three subsequent booster doses were subcutaneously injected at three week intervals and carried out through emulsifying the immunogen with incomplete Freund's adjuvant.

Subsequently, an immunization was carried out by having directly injected the immunogen, diluted in saline solution, into the peritoneal cavity. The ascitic fluid was obtained by intraperitoneal injection of SP20 myeloma cells.

### TEST ON SOLID PHASE

The solid phase of the test was obtained on PVC trays (Nunc), to which 100 µl of a P4 peptide solution, 10 µg/ml in carbonate buffer 50mM, pH 9.6, were added. Trays were incubated for 2 hours at 37°C.

Trays were saturated with 300 µl of a gelatin solution 2%, in Tris-HCl 0.1M, pH 7.5, for 1 hour at 37°C. The ascitic fluid was diluted 1:50; 1:100; 1:200 and 1:400 in a solution, comprising PBS, EDTA 0.03%, Triton-X100 0.2%, Tween 20 0.2% and BSA fract. V 3.2%, and incubated for 2 hours at 37°C. Trays were washed four times, using an ET-System Sorin automatic washer, with PBS/Tween 20 0.1%; and each well was added with 100 µl of anti mouse Ig antibodies, conjugated with HRP (horseradish peroxydase, Amersham), diluted 1:5000 in a buffer, comprising PBS and 0.1% of casein. Trays were incubated for 1 hour at 37°C. After four washing procedures with a solution of PBS/Tween 20, the reaction was detected by adding 100 µl of chromogen and substrate (Sorin DEIA KIT). The reaction was blocked after 30 min. by the addition of 200 µl of sulphuric acid 1N. The absorbance was read at 450 nm.

### ASCITIC FLUID PRECIPITATION WITH NA₂SO₄ 18%

The ascitic fluid (4.5 ml) was treated with Na₂SO₄ 18% (w/v 0.81 g) until completely dissolved. Then the mixture was incubated for 30 min. at 37°C and centrifuged at 4000 rpm for 15 min.

The resulting precipitate was collected using the PBS minimum volume and dialyzed overnight against PBS at 4°C.

### CHECK OF THE ACTIVITY OF THE ASCITIC FLUID FRACTION OBTAINED THROUGH PRECIPITATION WITH NA₂SO₄ 18%

The HAV adhered solid phases were supplied by Sorin Biomedica. 100 µl per well of fraction 18 of the ascitic fluid, diluted 1:20; 1:40; 1:80; 1:160; 1:320; 1:640 and 1:1280 in PBS, EDTA 0.03%, Triton-X100 0.2%, Tween 20 0.2% and BSA fract. V 3.2% solution, were incubated for 2 hours at 37°C. After four washings with PBS/Tween 0.1%, 100 µl per well of HRP conjugated, anti mouse Ig antibodies, 1:10000 diluted and a monoclonal antibody anti mouse K chain, were added at different dilutions in PBS, casein 0.1%. After four washings with a solution of PBS/Tween 0.1%, the reaction was detected by adding 100 µl of a chromogen and a substrate (Sorin DEIA KIT). The reaction was blocked after 30 min. by the addition of 200 µl of sulfuric acid 1N, and the 450 nm absorbance was readed.

### RESULTS

By using the methods as described previously under MATERIALS and METHODS, niniteen sublibraries were obtained, which showed an average representation of each amino acid of ± 7%, when submitted to amino acid analysis through deriving the amino acids with PITC before the column and quantifying by RP-HPLC.

The first nineteen sublibraries, of which only the amino acid N-terminal was known, were assayed with the HRP conjugated, 8F4H9B monoclonal antibody and, as control, with free peroxydase. Only one library was positive at the screening test. Once the reactive amino acid was identified, a further library synthesis was carried out, in order to avoid certain problems due to the reactive particle isolation. Accordingly, nineteen sublibraries were synthesized again, having each one the same N-terminal. Then, the sublibraries were tested again with the monoclonal antibody and with the free HRP, as control. The procedure as above was carried out until the whole sequence was defined. At each assay, a sublibrary was defined as positive when, after an OPD addition, showed the 492 nm highest absorbance, the development requested 2 hours and a twofold volume of sulfuric acid 1N, had to be used to block the reaction.

The identified sequence was as follows: DVMYLK (SEQ ID No.35). This sequence is not comprised within any linear portion of HAV codified proteins; therefore it represents a viral conformational epitope.

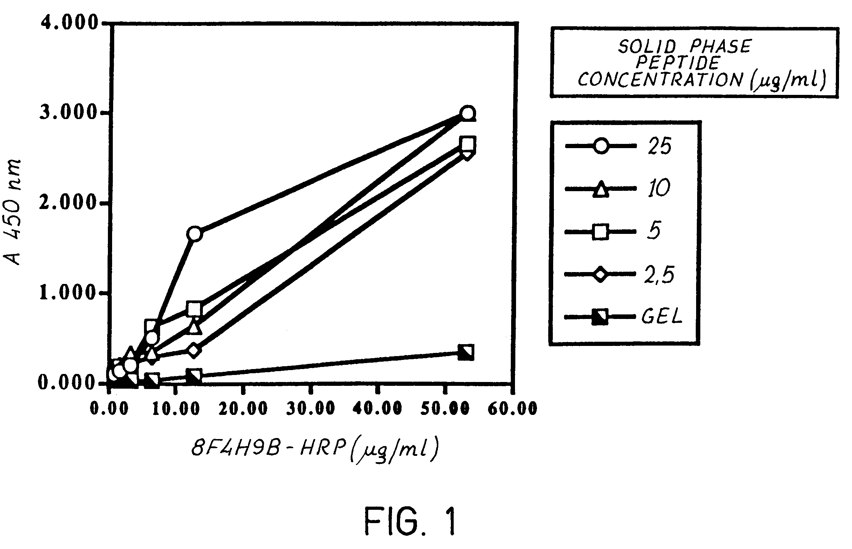

The corresponding peptide was synthesized in monomer form on solid phase with a spacer arm at C-terminal, comprising three glycine residues, and the obtained peptide, defined as PM, after having been adhered to a solid phase at different concentrations, was challenged with the 8F4H9B-HRP antibody (fig.1).

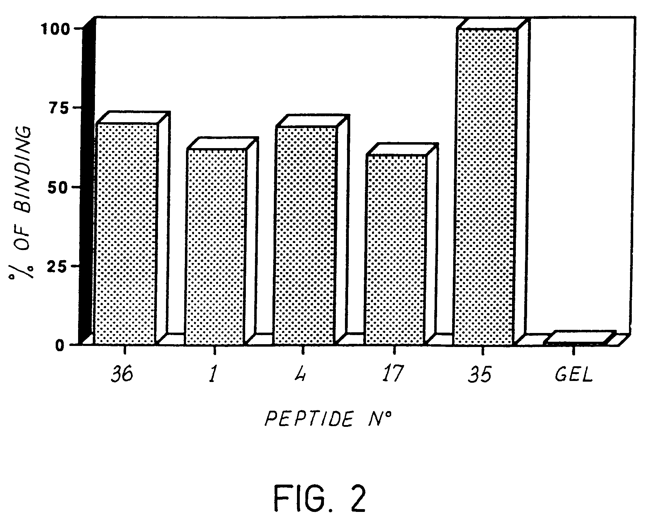

During the screening, some sublibraries resulted partially positive; i.e., they showed a little less 492 nm absorbance in respect of the highest reading. Therefore mutant peptides, corresponding to the various combinations, were synthesized. The 36 synthesized peptides are listed at Table 1, wherein PM is defined as peptide 35. Out of these peptides, only four, and namely, the peptides 36, 1, 4 and 17, reacted with the antibody, even if having shown a lesser reactivity than the PM peptide (Fig.2).

**Table 1**

| Mutant Peptides of the PM Peptide (SEQ ID NO. 35) | |
|---|---|
| 1. DVMWMH (SEQ ID No. 1) | 19. DFMYEH (SEQ ID No.19) |
| 2. DVMWMK (SEQ ID No. 2) | 20. DFMYEK (SEQ ID No.20) |
| 3. DVMYEH (SEQ ID No. 3) | 21. DFMYMH (SEQ ID No.21) |
| 4. DVMYEH (SEQ ID No. 4) | 22. DFMYMK (SEQ ID No.22) |
| 5. DVMYMH (SEQ ID No. 5) | 23. DFMKEH (SEQ ID No.23) |
| 6. DVMYMK (SEQ ID No. 6) | 24. DFMKLK (SEQ ID No.24) |
| 7. DVMKEH (SEQ ID No. 7) | 25. DFMKLH (SEQ ID No.25) |
| 8. DVMKLK (SEQ ID No. 8) | 26. DFMKMH (SEQ ID No.26) |
| 9. DVMKLH (SEQ ID No. 9) | 27. DFMKEK (SEQ ID No.27) |
| 10. DVMKMH (SEQ ID No.10) | 28. DFMKMK (SEQ ID No.28) |
| 11. DVMKEK (SEQ ID No.11) | 29. DFMWEH (SEQ ID No.29) |
| 12. DVMKMK (SEQ ID No.12) | 30. DFMWLK (SEQ ID No.30) |
| 13. DVMWEH (SEQ ID No.13) | 31. DFMWLH (SEQ ID No.31) |
| 14. DVMWLK (SEQ ID No.14) | 32. DFMWEK (SEQ ID No.32) |
| 15. DVMWLH (SEQ ID No.15) | 33. DFMYLH (SEQ ID No.33) |
| 16. DVMWEK (SEQ ID No.16) | 34. DFMYLK (SEQ ID No.34) |
| 17. DFMWMH (SEQ ID No.17) | 35. DVMYLK (SEQ ID No.35) |
| 18. DFMWMK (SEQ ID No.18) | 36. DVMYLH (SEQ ID No.36) |

However, the PM reactivity against the 8F4H9B antibody was not optimal, probably because such small peptides had some problems to adhere to the solid phase.

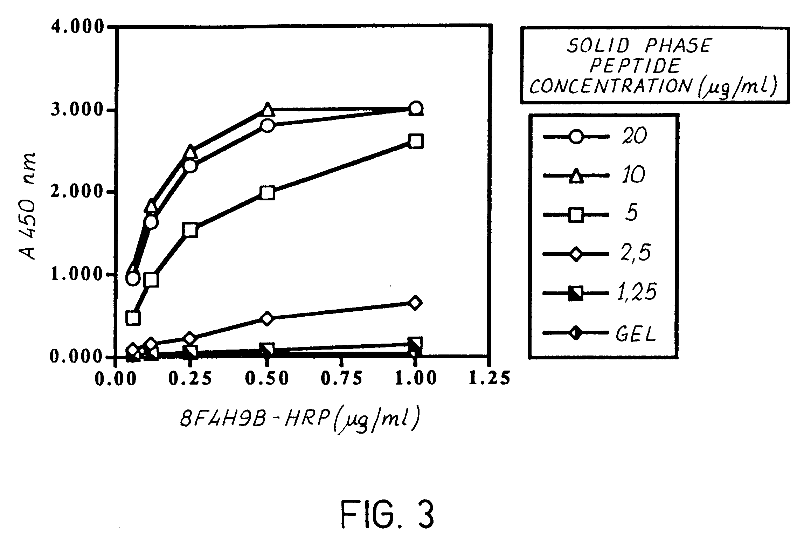

In order to increase this reactivity, a tetramer form PM peptide (P4) was synthesized, using a lysine core (MAP System), having the following formula: 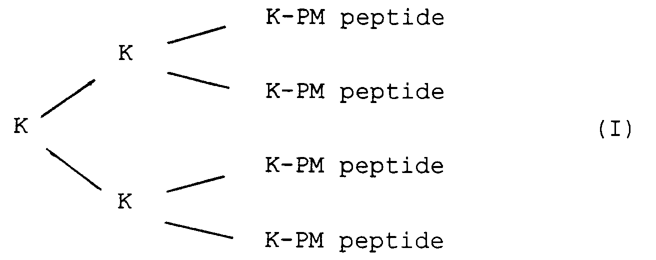
The peptide in such form showed a marked reactivity increase in respect of the monomer peptide (fig.3); actually, in order to obtain the same PM reactivity, only one fiftieth of the 8F4H9B antibody could be used.

In order to exclude any unspecific interaction, the reactivity of the 8F4H9B monoclonal antibody with a lysine core was compared in respect of the lysine core, the anchored peptide-free and a unrelated tetramer peptide reactivities. The P4 reactivity is highly stronger than the reactivities of the unrelated peptide and of the antibody bond-free lysine core. The binding specificity was confirmed by an assay, wherein the free peroxydase was shown to be not reactive against the P4 peptide.

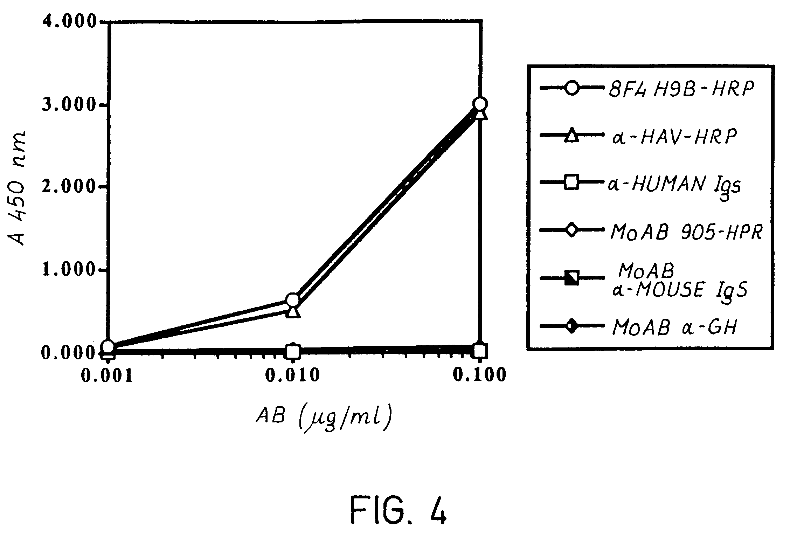

When the P4 peptide was tested against human HAV anti polyclonal antibodies, the same reactivity, as against the 8F4H9B-HRP monoclonal antibody, was detected. Other not peroxydase conjugated mono- or poly-clonal not related antibodies, did not bind to the peptide (fig.4).

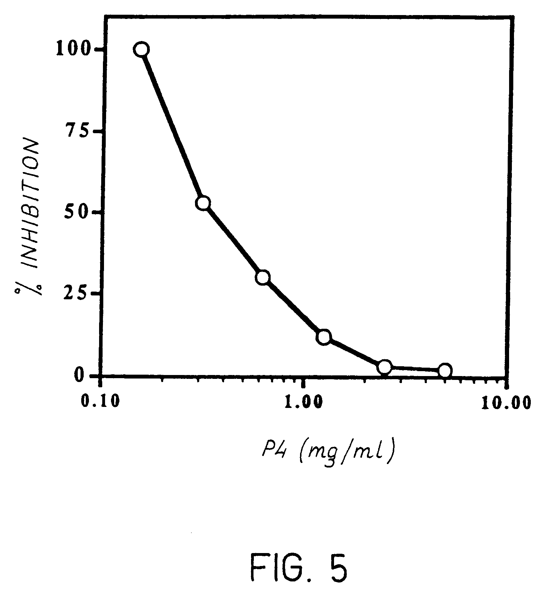

Inhibition tests were carried out by using trays, wherein HAV was adhered to the solid phase. The 8F4H9B antibody at a constant concentration was added to these trays, while the P4 peptide was added at decreasing concentrations. As shown in fig.5, the peptide inhibited the antibody to bind to the virus and, under an appr. 5 mg/ml concentration, a total inhibition occurred.

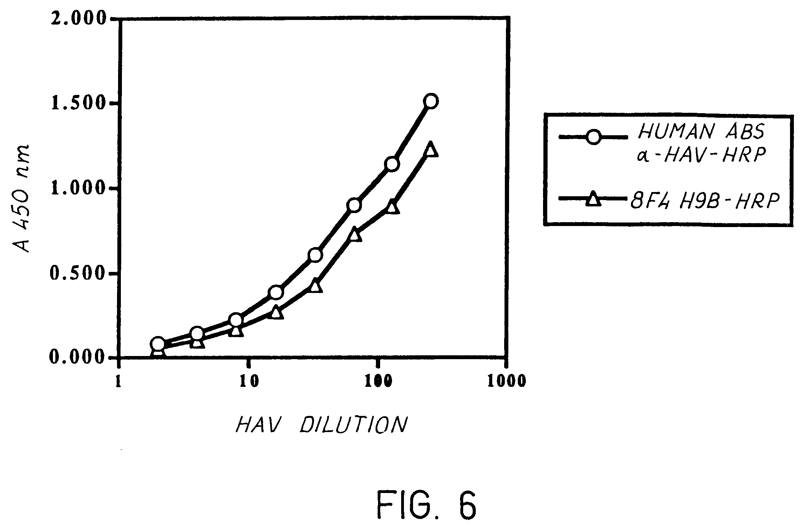

The availability of HAV containing supernatants allowed to perform a modification of the test as above. The 8F4H9B antibody and human anti-HAV polyclonal antibodies, at fixed concentrations, were added to the trays, to which the peptides adhered, in the presence of diminishing concentrations of HAV containing culture medium. As shown in fig.6, by decreasing the virus concentration, a gradual increase of the reactivity between either the 8F4H9B antibody, or the human anti-HAV polyclonal antibodies, and the P4 peptide, was detected.

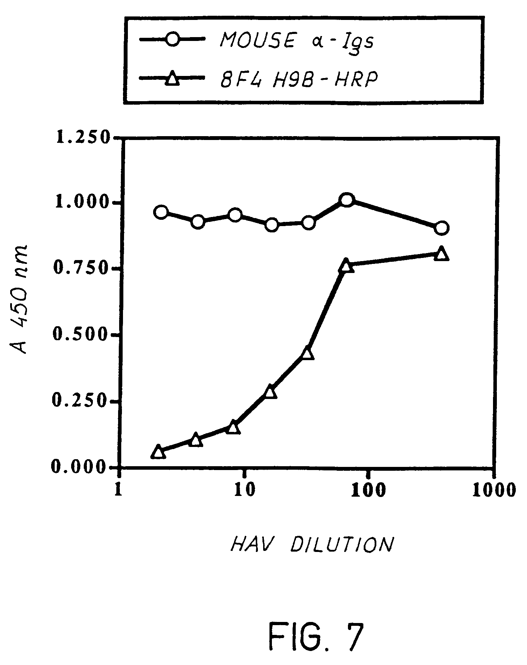

These findings allowed to establish that the peptide interacted with the paratope of the specific antibody. The components of the culture medium showed not to interfere aspecifically with the reaction; actually, by having used trays, to which an unrelated monoclonal antibody adhered, and incubated the culture medium with a anti mouse Ig polyclonal antibody under the same conditions, the culture medium did not show to inhibit the binding (fig.7).

In order to further exclude the possibility of some interaction between the peptide P4 and either the peroxydase, or a formed epitope at the junction of the HRP and the antibody fraction, a unconjugated 8F4H9B monoclonal antibody was tested against the peroxydase. In this case a less strong binding occurred; however the same result was obtained also on HAV containing trays. This is probably due to the fact that the virus and peptide amounts, that adhered to the solid phase, were limiting under these conditions.

Therefore it was possible to conclude that an extremely specific binding occured between the antibody and the peptide, as well as the interaction site between the antibody and the peptide is the same interaction site of virus binding.

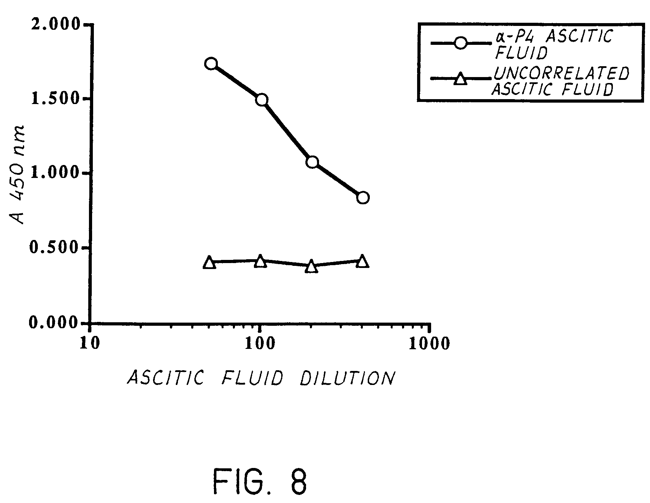

In order to check whether the P4 peptide was able to induce an immunogenic response, the ascitic fluid, that was picked up following the BALB/C mice immunization with the hemocyanine conjugated, and unconjugated tetramer peptide, was tested with the peptide adhering to the solid phase, and a specific reactivity at the different dilutions was detected (fig.8).

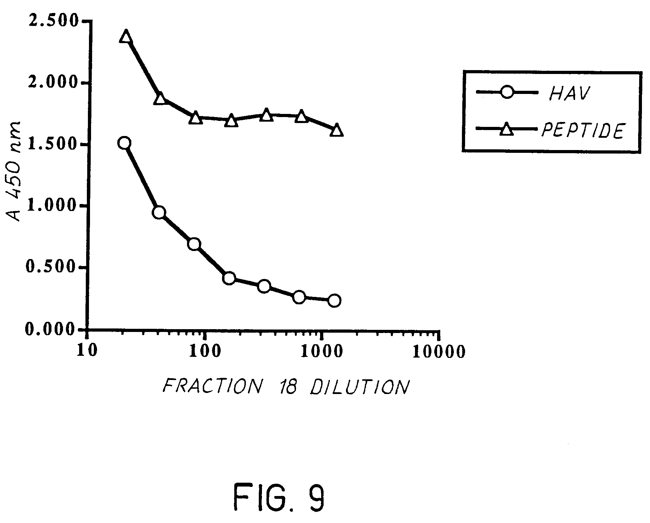

In order to improve the ascitic fluid reactivity and to prevent any unspecific reactivity due to some ascitic fluid component, a precipitation with Na₂SO₄ 18%, was carried out. The precipitate was collected with PBS and, after having been dialyzed, was tested with the peptide and HAV adhering to the solid phase. Fig.9 shows the concerned reactivity when HRP conjugated and 1:10000 diluted goat anti mouse Ig immunoglobulins were used as labelling system.

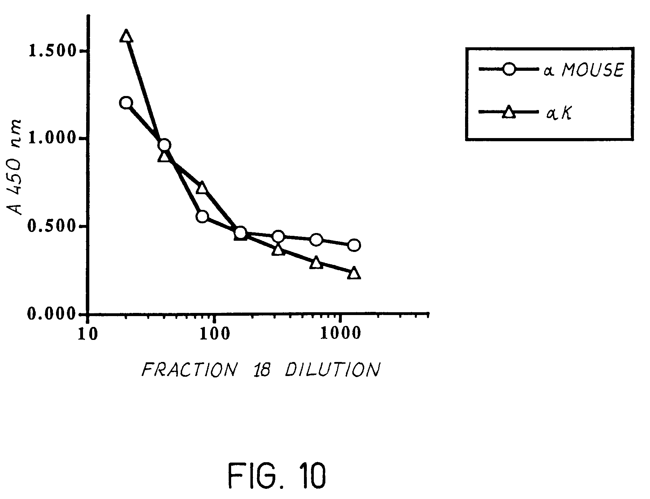

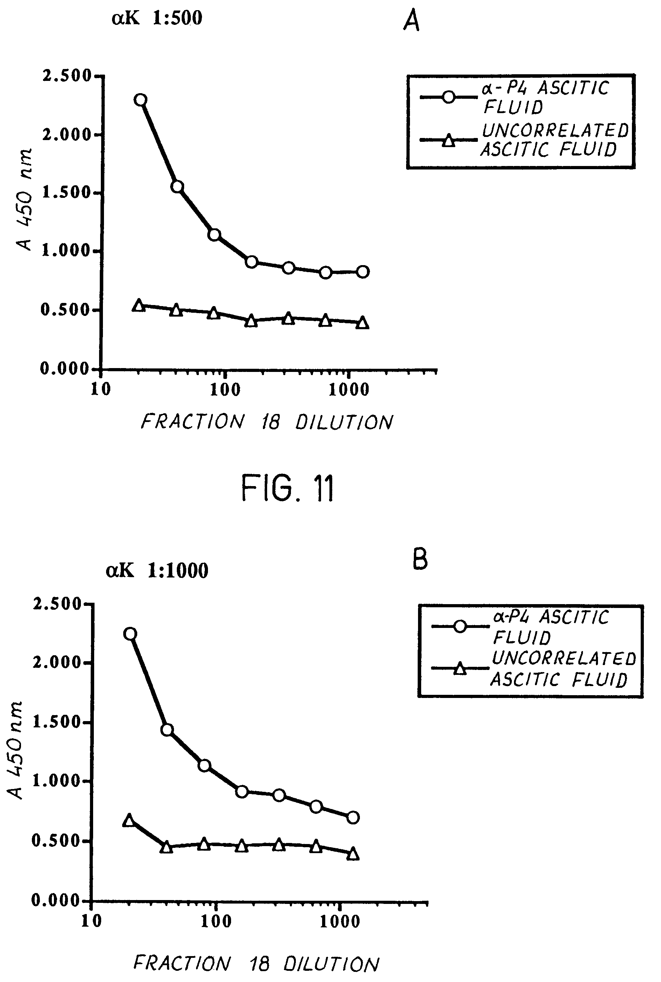

In order to improve the HAV reactivity against the fraction 18 of the anti-peptide ascitic fluid, a labelling system was used, comprising an anti mouse K chain monoclonal. Fig.10 shows the reactivity increase. In order to check the specific nature of the binding between the virus and the anti-peptide fraction 18 of the ascitic fluid, tests were carried out, using the fraction 18 of an ascitic fluid, that was picked up from mice immunized with an unrelated peptide and, as labelling system, the anti mouse K chain monoclonal antibody, previously tested at various concentrations (fig.11). These tests confirmed the specific nature of the binding between HAV and the anti-peptide fraction 18 of the ascitic fluid.

### OPTIMIZING THE HAV REACTIVE AMINO ACID SEQUENCE

The P4 peptide ability to increase its reactivity through an elongation with 3 or 6 amino acids was tested. Accordingly, new libraries, having the formula as follows: AA₁-AA₂-AA₃-DVMYLK-GGG-PAM were synthesized, wherein the sequence DVMYLK (SEQ ID No. 35) was kept constant, while three positions were random analyzed.

After having identified the reactive sequence: MWL-DVMYLK-GGG (SEQ ID No.37), libraries as follows were synthesized: AA₁-AA₂-AA₃-MWL-DVMYLK-GGG-PAM, allowing to identify the following sequence: ISGMWLDVMYLK-GGG (SEQ ID No.46).

Monomer and tetramer form peptides were obtained through solid phase synthesis, which were tested for reactivity with the 8F4H9B monoclonal antibody.

### PEPTIDE SUBLIBRARY SYNTHESIS

The two synthesized peptide libraries showed the following general formulas:
1. AA₁-AA₂-AA₃-DVMYLK-GGG-PAM; and
2. AA₁-AA₂-AA₃-MWL-DVMYLK-GGG-PAM
0.6 g of Boc-Gly-PAM resin with 0.075 mmol/g function level, were suspended in DCM, filtered and treated with a 1:1 mixture of TFA/DMC under low stirring for 30 min. at room temperature in order to release the Boc group. The resin was five time washed with DCM and treated with a 1:10 mixture of TEA/DCM under low stirring for 10 min. at room temperature. After having been three time washed with DCM and once with DMF, the resin was reacted with a 5X excess of Fmoc-Gly-OPfp in HOBT, 0.33 M/DMF, under stirring at room temperature, until the reaction was completed, that was monitored through the Kaiser's Test. When the coupling was obtained, the resin was washed three times with DMF, incubated with a solution of piperidine 20%, in DMF for 20 min. at room temperature, washed three times with DMF, twice with methanol, three times with DMF and then coupled with the amino acids as follows: Fmoc-Gly-OPfp; Fmoc-Lys(tBoo)-OPfp; Fmoc-Leu-OPfp; Fmoc-Tyr(tBu)-OPfp; Fmoc-Met-OPfp; Fmoc-Val-OPfp and Fmoc-Asp(OtBu)-OPfp, using the same procedure as to sinthesize the first library, as well as with the following amino acids: Fmoc-Gly-OPfp; Fmoc-Lys(tBoc)-OPfp; Fmoc-Leu-OPfp; Fmoc-Tyr(tBu)-OPfp; Fmoc-Met-OPfp; Fmoc-Val-OPfp, Fmoc-Asp(OtBu)-OPfp; Fmoc-Leu-OPfp; Fmoc-Trp-OPfp and Fmoc-Met-OPfp, as to synthesize the second library.

### SYNTHESIS OF THE RANDOM PORTION

### AA₃ POSITION

After having synthesized the constant portion, the amino group group of the resin was deprotected by a solution of piperidine 20%, in DMF at room temperature; washed with DMF; suspended in a 60:40 mixture of DCM/DMF and subdivided in 19 fractions at room temperature under stirring. Each resin fraction was added with a different amino acid in 5X excess of HOBT, 0.33 M/DMF. All used amino acids were protected at the alpha position of the amino group with a Fmoc group, preactivated at carboxyl group as pentafluorophenyl esters and as hydroxydihydrobenzotriazine esters when serine and threonine were concerned. The side chain protecting groups were as follows: Mtr for arginine; Trt for asparagine, glutamine and histidine; tBu for tyrosine, serine and threonine; and OtBu for aspartic and glutamic chains.

The reactions were completed at room temperature under stirring (under Kaiser's test monitoring). Aliquots were washed with DMF, collected together and treated with a mixture, comprising piperidine 20%, in DMF at room temperature under rotation. After having been washed with DMF, an amount equal to one fiftieth of each portion was picked up and maintained at 4°C, in order to carry out the interactive synthesis of the subsequent libraries. The remaining resin was collected, washed with DMF, treated with piperidine 20%, in DMF for 20 min. at room temperature, and washed twice with CH₃OH and three times with DMF.

### POSITION AA₂

The resin was suspended in the mixture 60:40 DCM/DMF, stirred for 20 min. at room temperature, subdivided in 19 aliquots and submitted at the same procedure as above, except the resin picking up from each portion, that was one third in this case.

### POSITION AA₁

The method previously described was also used to couple the last amino acid. The aliquots, after the coupling reactions, were kept separated. The N-terminal amino group was released using piperidine 20%, in DMF for 20 min. at room temperature; washings were carried out using DMF, glacial acetic acid, t-amyl alcohol and ethyl ether. The portions were vacuum dried for 2 hours. Then the sublibraries were subjected to a cleavage with a mixture, comprising TFA 85%, EDT 2.5%, phenol 5%, thioanizole 5%, and H₂O 2.5%, overnight at room temperature, in order to remove the protecting groups on the side chains.

The following libraries were synthesized by adding the N-terminal amino acids, identified through the screening, to the picked up amounts of each portion at each coupling step.

The resulting library probing, the identified peptide synthesis and the ELISA assays were carried out as described in the previous chapters.

### RESULTS

### AA₁-AA₂-AA₃-DVMYLK-GGG LIBRARY

The library screening with the method as herebelow, led to identify the peptides, sequences of which are indicated in the Table 2 herebelow.

**Table 2**

| |
|---|
| 1. MWL-DVMYLK(SEQ ID No.37) |
| 2. MLL-DVMYLK(SEQ ID No.38) |
| 3. MVL-DVMYLK(SEQ ID No.39) |
| 4. MWH-DVMYLK(SEQ ID No.40) |
| 5. MLH-DVMYLK(SEQ ID No.41) |
| 6. MVH-DVMYLK(SEQ ID No.42) |

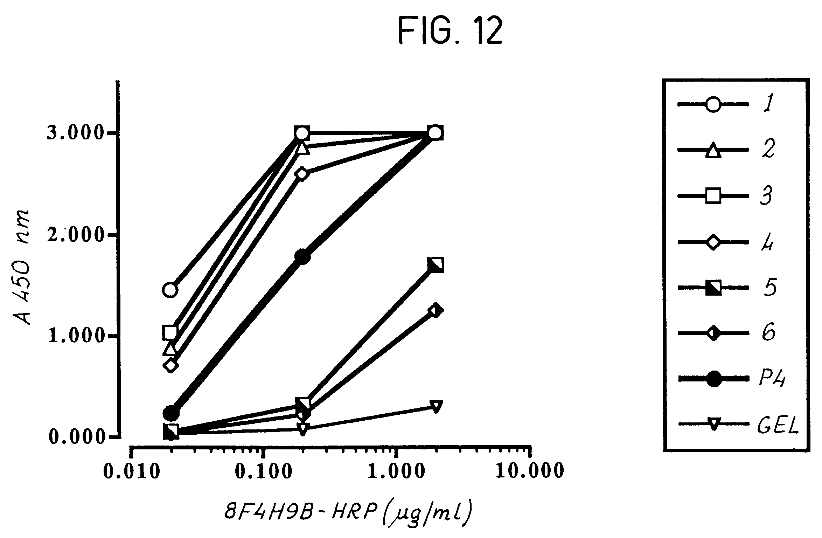

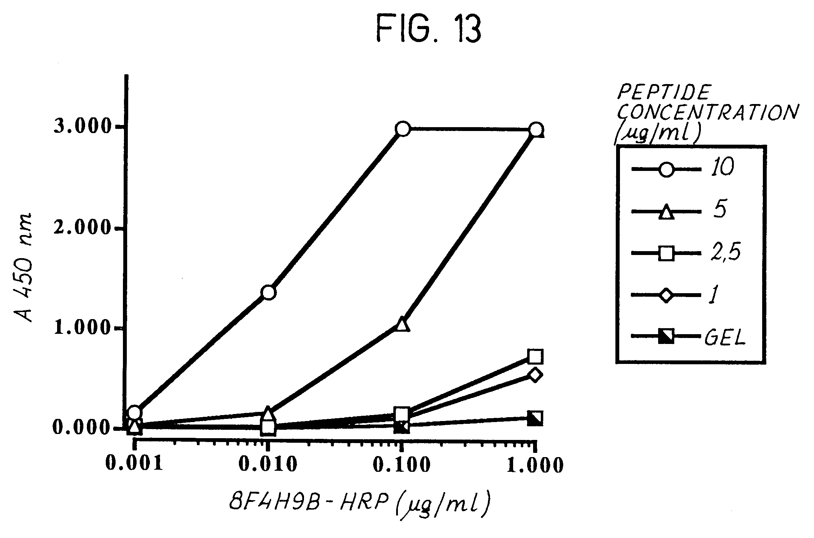

The peptides were synthesized in tetramer form and assayed, when adhering to the solid phase at various concentrations, against the 8F4H9B monoclonal antibody and purified human anti-HAV Igs. The peptide 1 showed a higher reactivity than the DVMYLK-GGG P4 tetramer peptide, that was identified originally (fig.12 and 13). The peptide 1 was further assayed with unrelated mono- and polyclonal antibodies in order to check the binding specificity (fig.11).

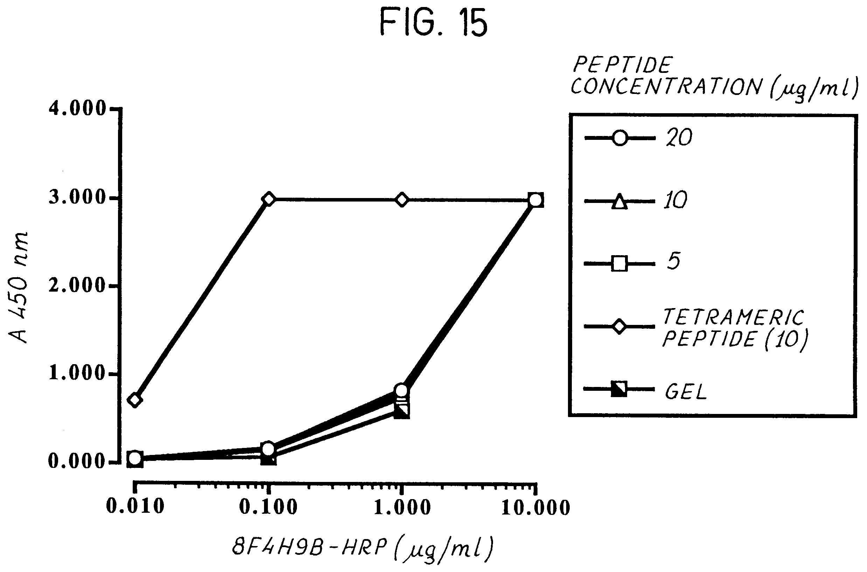

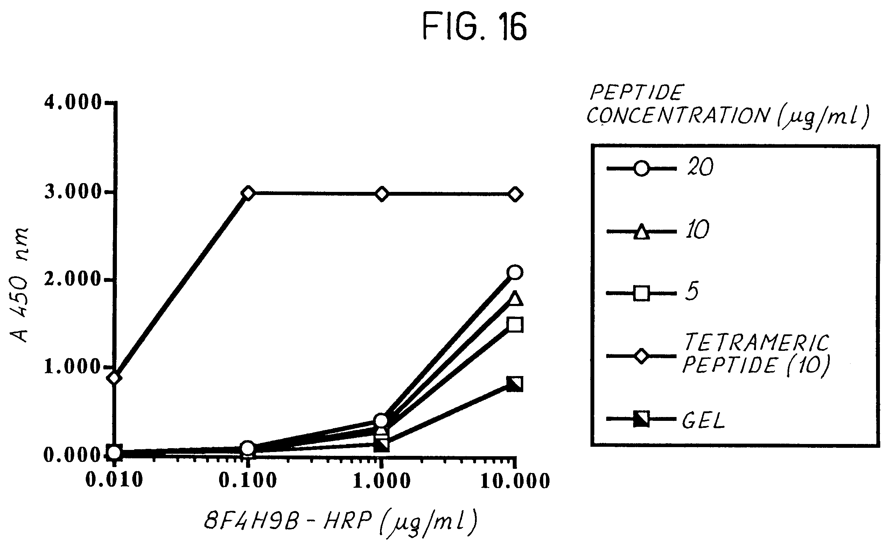

Then the peptide 1 was synthesized in monomer form with the following sequence: MWLDVMYLKGGGC and tested on solid phase, using the following solid phase adhering conditions:
- carbonate buffer 50 mM, pH 9.6, for 2 hours at 37°C (fig.15);
- carbonate buffer 50 mM, pH 9.6, overnight at 4°C (fig.16); and
- solution of trifluoroethanol 50 %, and H₂O over-night at room temperature.

The peptide in monomer form showed the highest reactivity when adhered to the solid phase, after having been diluted into a solution of trifluoroethanol 50 % in H₂O.

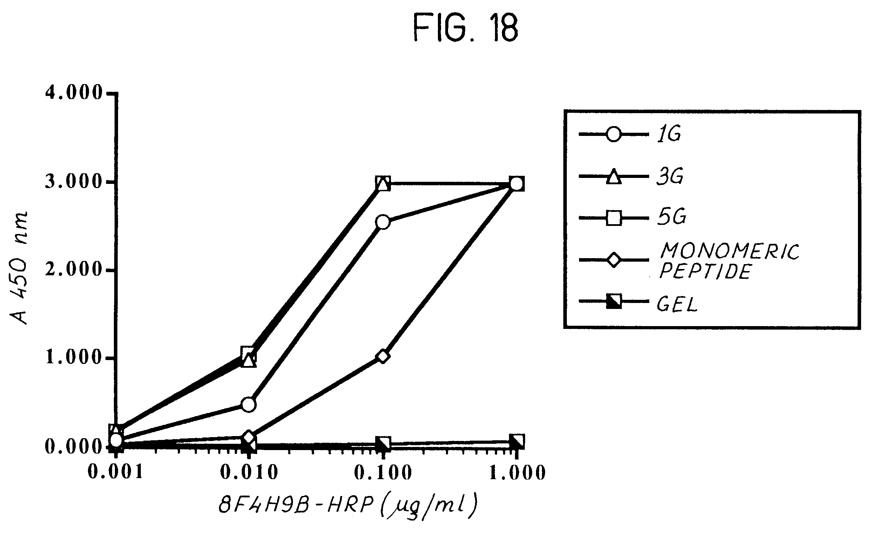

In order to further increase the monomer peptide reactivity, the peptides as follows were synthesized:
1G MWLDVMYLK-G-MWLDVMYLK-GGG-C;
3G MWLDVMYLK-GGG-MWLDVMYLK-GGG-C; and
5G MWLDVMYLK-GGGGG-MWLDVMYLK-GGG-C
The peptides were assayed on solid phase (fig.18) and showed a higher reactivity than the single monomer peptide, that could be compared with the tetramer form peptide reactivity.

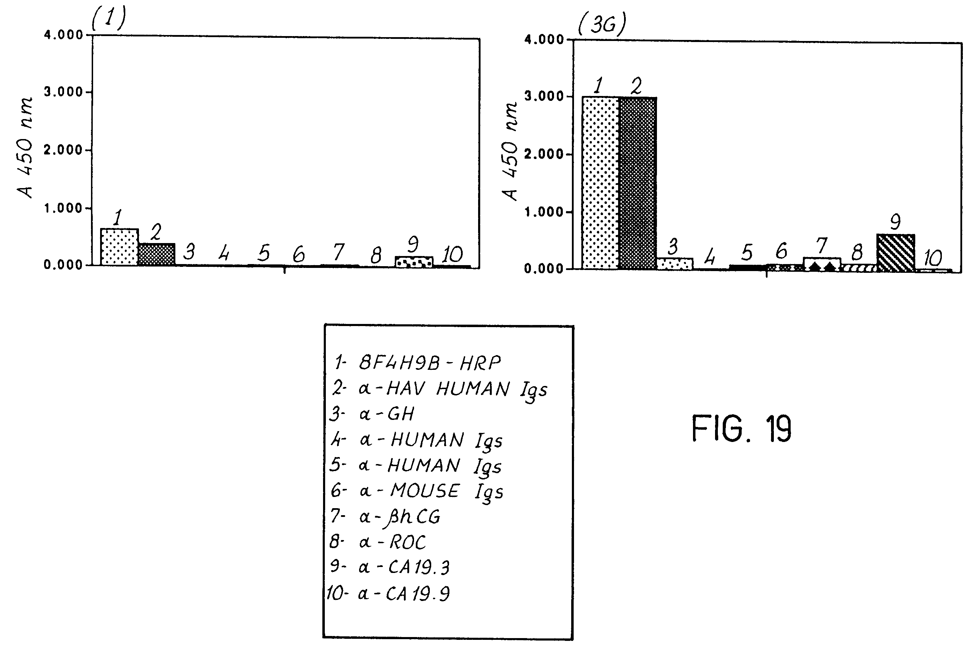

All peptides were assayed against unrelated mono- and polyclonal antibodies in order to check the specific nature of the binding to the 8F4H9B monoclonal antibody (fig.19).
AA₁-AA₂-AA₃-MWL-DVMYLK-GGG-PAM LIBRARY
The AA₁-AA₂-AA₃-MWL-DVMYLK-GGG-PAM library screening identified the sequences as follows:
1A. ISA-MWLDVMYLK (SEQ ID No.43)
1B. TSA-MWLDVMYLK (SEQ ID No.44)
1C. NSA-MWLDVMYLK (SEQ ID No.45)
1D. ISG-MWLDVMYLK (SEQ ID No.46)
1E. TSG-MWLDVMLYK (SEQ ID No.47)
1F. NSG-MWLDVMYLK (SEQ ID No.48).

The peptides were synthesized in monomer form and assayed at various concentrations on solid phase against the 8F4H9B monoclonal antibody, as well as purified human anti-HAV Igs.

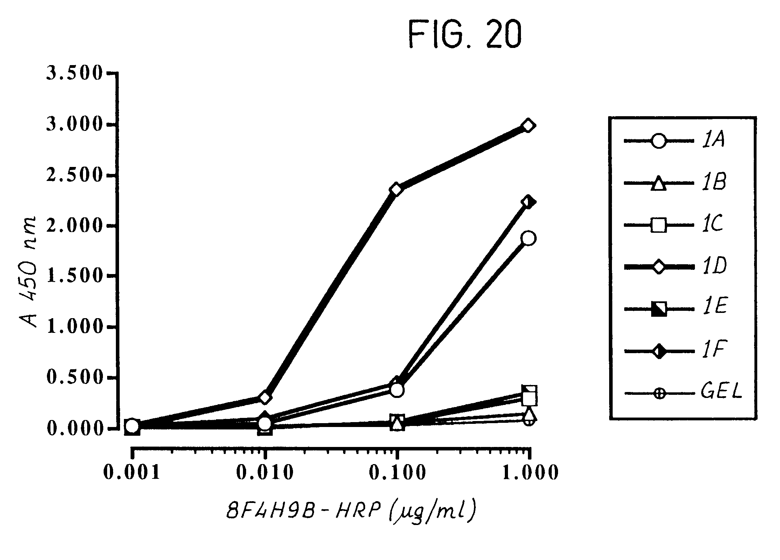

Only the 1D peptide, having the following sequence: ISG-MWLDVMYLK-GGG showed a reactivity, that could be compared with the previously synthesized peptides (fig.20).

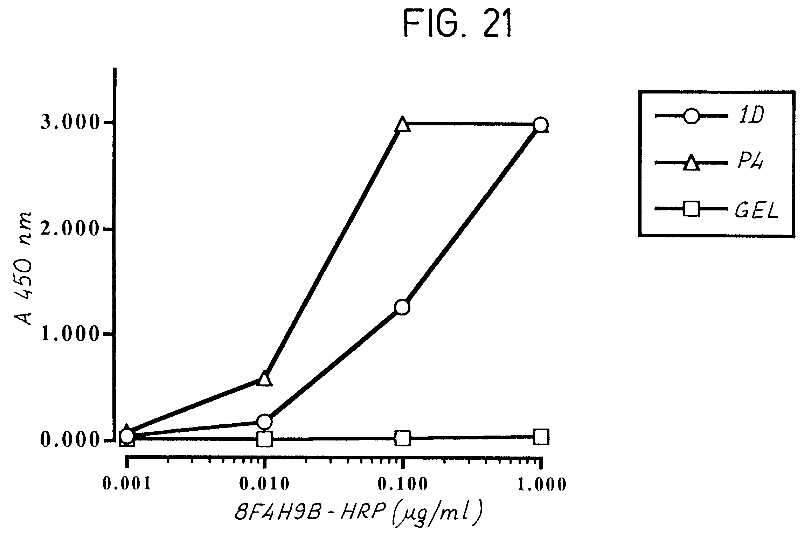

The peptide was synthesized in tetramer form and compared against the 1 peptide as per previous chapther, and showed a lesser reactivity (fig.21).

### REFERENCES

(1) Erickson, B.W, and Merrifield, R.B.; "The proteins", Neurath, H. and Hill, R.L. Ed., vol.2, 3rd ed., Academic Press, New York, 255-527 (1976).
(2) Kaiser, E., Colescott, R.L., Bossinger, C.D. and Cook, P.I.; Anal. Biochem., 34, 595-598 (1970).
(3) Tam, J.P.; "Proc. Nat. Acad. Sci. USA", 85, 5409-5420 (1988).
(4) Bidlingmeyer, B.A., Cohen, S.A. and Tarvin, S.M., "T.L. Chromatogr.", 336, 93-104 (1984).

## Claims

1. A peptide with antigenic activity against HAV, having an amino acid sequence that cannot be identified from the amino acid sequence of HAV codified proteins, at least comprising the sequence, as identified as SEQ ID No. 35.

2. A peptide having an antigenic activity against HAV according to claim 1, comprising the sequence, as identified as SEQ ID No. 37.

3. A peptide having an antigenic activity against HAV according to claim 2, comprising the sequence, as identified as SEQ ID No. 46.

4. A peptide having an antigenic activity against HAV according to any of previous claims, comprising a spacer arm.

5. A peptide having an antigenic activity against HAV according to claim 4, wherein said spacer arm at least comprises three glycine residues, that are bound either to the peptide C-terminal or N-terminal by means of a peptidic binding.

6. A peptide having an antigenic activity against HAV according to any of previous claim, wherein said peptide is as a linear monomer form.

7. A peptide having an antigenic activity against HAV according to claim 6, wherein said amino acid sequence is repeated more than once.

8. A peptide having an antigenic activity against HAV according to claim 7, wherein said amino acid sequence is twice repeated and spaced at least by a glycine residue.

9. A peptide having an antigenic activity against HAV according to claim 8, wherein said glycine residues are three or five.

10. A peptide having an antigenic activity against HAV according to any of claims from 1 to 5, wherein said peptide ss as a not linear polymer form.

11. A peptide having an antigenic activity against HAV according to claim 10, wherein said peptide has a tetramer form, according to the following formula: 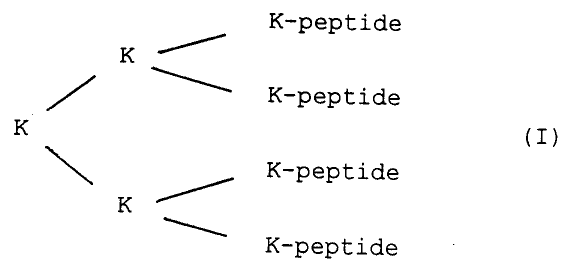 wherein K is a lysine residue.

12. A composition comprising the peptide according to any of previous claims, to be used as specific reactant for preparing diagnostic kits, that are able to identify anti-HAV antibodies from samples.

13. An anti-HAV immunogenic composition comprising a peptide according to any of claims from 1 to 11, to be used as a vaccine or as starting material to prepare anti-HAV immune sera.
